# EUROPEAN PATENT APPLICATION

(11) **EP 0 861 902 A1**
(43) Date of publication of application: **02.09.1998**
(21) Application number: 97940364.9
(22) Date of filing: 12.09.1997
(51) Int. Cl.: C12P 19/00, C12P 19/32

(54) **PROCESSES FOR PRODUCING SUGAR NUCLEOTIDES AND COMPLEX CARBOHYDRATES**

(30) Priority: 13.09.1996 JP 243120/96; 28.10.1996 JP 285065/96
(71) Applicant: Kyowa Hakko Kogyo Co., Ltd., Chiyoda-ku, Tokyo 100 (JP)
(72) Inventor: KOIZUMI, Satoshi, Machida-shi, Tokyo 194 (JP); KAWANO, Hisaji, Saba-gun, Yamaguchi 747-02 (JP); KINO, Kuniki, Hofu-shi, Yamaguchi 747 (JP); OZAKI, Akio, Machida-shi, Tokyo 194 (JP)
(74) Representative: VOSSIUS & PARTNER
(86) International application number: JP9703225
(87) International publication number: WO9811247

(57) **Abstract**

This invention relates to a process for producing a complex carbohydrate, which comprises: selecting, as enzyme sources, a culture broth of a microorganism capable of producing a sugar nucleotide from a nucleotide precursor and a sugar, or a treated product of the culture broth, and a culture broth of a microorganism or animal cell capable of producing a complex carbohydrate from a sugar nucleotide and a complex carbohydrate precursor, or a treated product of the culture broth; carrying out an enzyme reaction in an aqueous medium containing the enzyme sources, the nucleotide precursor, the sugar and the complex carbohydrate precursor to form and accumulate the complex carbohydrate in the aqueous medium; and recovering the complex carbohydrate from the aqueous medium, and a process for producing a sugar nucleotide, which comprises selecting, as an enzyme source, a culture broth of a microorganism capable of producing a sugar nucleotide from a nucleotide precursor and a sugar, or a treated product of the culture broth; carrying out an enzyme reaction in an aqueous medium containing the enzyme source, the nucleotide precursor and the sugar to form and accumulate the sugar nucleotide in the aqueous medium; and recovering the sugar nucleotide from the aqueous medium.

## Description

### TECHNICAL FIELD

This invention relates to a process for producing a complex carbohydrate which is useful for protection against infection of bacteria, viruses, and the like, application to cardiovascular disorders and immunotherapy, and a process for producing a sugar nucleotide which is important as a synthetic substrate of the complex carbohydrate.

### BACKGROUND ART

Examples of the known methods for producing sugar nucleotides include: 1) chemical synthetic methods (*Adv. Carbohydr. Chem. Biochem.*, 28, 307 (1973), *Bull. Chem. Soc. Japan*, 46, 3275 (1973), *J. Org. Chem*., 57, 146 (1992), *Carbohydr. Res.*, 242, 69 (1993)); 2) production methods using an enzyme (*J. Org. Chem.*, 55, 1834 (1992), *J. Org. Chem.*, 57, 152 (1992), *J. Am. Chem. Soc*., 110, 7159 (1988), Japanese Published National Publication No. 508413/95, Japanese Published National Publication No. 5000248/95, WO 96/27670); 3) methods using microbial cells such as yeast and the like (Japanese Examined Patent Application No. 2073/70, Japanese Published Examined Patent Application No. 40756/71, Japanese Published Examined Patent Application No. 1837/72, Japanese Published Examined Patent Application No. 26703/72, Japanese Published Examined Patent Application No. 8278/74, Japanese Published Unexamined Patent Application No. 268692/90); and 4) an extraction method from microbial cells of halo-tolerant yeast (Japanese Published Unexamined Patent Application No. 23993/96).

However, the method 1) requires expensive materials (for example, morpholidate derivative of uridine-5'-monophosphate (referred to as "UMP" hereinafter), sugar phosphate, *etc*.); the method 2) requires expensive materials (for example, UMP, uridine-5'-diphosphate (referred to as "UDP" hereinafter), uridine-5'-triphosphate (referred to as "UTP" hereinafter), adenosine-5'-triphosphate (referred to as "ATP" hereinafter), phosphoenolpyruvate, sugar phosphate, *etc*.), and various enzymes (for example, pyruvate kinase, *etc*.); and the method 3) requires drying treatment of yeast cells and expensive materials (for example, UMP, *etc*.). Including the method 4), all of the above-mentioned methods use expensive uridine nucleotides, sugar phosphates, and the like, or have a difficulty in effecting large scale production from the operational point of view, so that an industrial scale production method of sugar nucleotides has not so far been established.

Examples of the known method for producing complex carbohydrates include 1) chemical synthetic methods (*Method in Enzymol*., 247, 193 (1994), *Angew. Chem. Int. Ed. Engl.*, 21, 155 (1988), *Carbohydr. Res.*, 211, c1 (1991)); 2) methods in which a hydrolase is used (*Anal. Biochem.*, 202, 215 (1992), *Trends Biotechnol.*, 6, 256 (1988)); and 3) methods in which a glycosyltransferase is used (Japanese Published Unexamined Patent Application No. 79792/95, Japanese Published National Publication No. 500248/95, Japanese Examined Patent Application No. 82200/93, WO 94/25614).

The introduction of protecting groups is essential for stereo-selective synthesis in the method 1). The yield and selectivity are not sufficient in the method 2). Expensive materials are necessary in the method 3). These methods 1) to 3) have not been established as industrial production methods of complex carbohydrates.

It has been reported that UMP is produced in a microorganism belonging to the genus *Corynebacterium* when orotic acid is added (*Amino Acid, Nucleic Acid,* 23, 107 (1971)).

### DISCLOSURE OF THE INVENTION

An object of the present invention is to provide a process for producing a complex carbohydrate which is useful for protection against infection of bacteria, viruses, and the like, application to cardiovascular disorders and immunotherapy, and a process for producing a sugar nucleotide which is important as a substrate for synthesizing the complex carbohydrate at a low cost and efficiently.

The inventors of the present invention have conducted intensive studies on a method for producing sugar nucleotides by using microorganisms and have found as the results that a sugar nucleotide can be produced when a nucleotide precursor and a sugar are added to a culture broth during culturing of a microorganism and that a complex carbohydrate can be produced using the sugar nucleotide, thereby resulting in the accomplishment of the present invention.

The present invention provides a process for producing a sugar nucleotide, which comprises: selecting, as an enzyme source, a culture broth of a microorganism capable of producing a sugar nucleotide from a nucleotide precursor and a sugar, or a treated product of the culture broth; carrying out an enzyme reaction in an agueous medium containing the enzyme source, the nucleotide precursor and the sugar to form and accumulate the sugar nucleotide in the agueous medium; and recovering the sugar nucleotide from the agueous medium.

Furthermore, the present invention provides a process for producing a complex carbohydrate, which comprises: selecting, as enzyme sources, a culture broth of a microorganism capable of producing a sugar nucleotide from a nucleotide precursor and a sugar, or a treated product of the culture broth, and a culture broth of a microorganism or animal cell capable of producing a complex carbohydrate from a sugar nucleotide and a complex carbohydrate precursor, or a treated product of the culture broth; carrying out an enzyme reaction in an aqueous medium containing the enzyme sources, the nucleotide precursor, the sugar and the complex carbohydrate precursor to form and accumulate the complex carbohydrate in the agueous medium; and recovering the complex carbohydrate from the aqueous medium.

According to the present invention, a novel production process of a sugar nucleotide and a novel production process of a complex carbohydrate making use of the sugar nucleotide production process can be provided, which are characterized in that 1) expensive starting materials (for example, uridine nucleotides, sugar phosphates, *etc*.) are not required, and inexpensive nucleotide precursor (e.g., orotic acid, *etc*.) and a sugar can be used as the starting materials; 2) addition of expensive phosphoenolpyruvate and pyruvate kinase is not necessary in converting UDP into UTP; and 3) a process for the isolation of enzymes is not necessary.

The present invention will be described below in detail.

With regard to the microorganism which can be used for producing the sugar nucleotide according to the present invention, any microorganism, such as a microorganism belonging to yeast, can be used, with the proviso that it has an ability to produce a sugar nucleotide from a nucleotide precursor and a sugar. Specific examples include microorganisms belonging to the genera *Saccharomyces*, *Candida*, *Pichia*, *Torulopsi*s, *Debaryomyces*, *Zygosaccharomyces*, *Kluyveromyces*, *Hansenula* and *Brettanomyces*. Among these, preferred microorganisms belonging to the genus *Saccharomyces* include *Saccharomyces cerevisiae*, *etc*.; preferred microorganisms belonging to the genus *Candida* include *Candida utilis*, *Candida parapsilosi*s, *Candida krusei*, *Candida versatilis*, *Candida lipolytica*, *Candida zeylanoides*, *Candida guilliermondii*, *Candida albicans*, *Candida humicola*, *etc*.; preferred microorganisms belonging to the genus *Pichia* include Pichia *farinosa*, *Pichia ohmeri*, *etc*.; preferred microorganisms belonging to the genus *Torulopsis* include *Torulopsis candida*, *Torulopsis sphaerica*, *Torulopsis xylinus*, *Torulopsis famata*, *Torulopsis versatilis*, *etc*.; preferred microorganisms belonging to the genus *Debaryomyces* include *Debaryomyces subglobosus*, *Debaryomyces cantarellii*, *Debaryomyces globosus*, *Debaryomyces hansenii*, *Debaryomyces japonicus*, *etc*.; preferred microorganisms belonging to the genus *Zygosaccharomyces* include *Zygosaccharomyces rouxii*, *Zygosaccharomyces bailii*, etc.; preferred microorganisms belonging to the genus *Kluyveromyces* include *Kluyveromyces marxianus*, *etc*.; preferred microorganisms belonging to the genus *Hansenula* include *Hansenula anomala*, *Hansenula jadinii*, *etc*.); and preferred microorganisms belonging to the genus *Brettanomyces* include *Brettanomyces lambicus*, *Brettanomyces anomalus*, *etc*.

In addition, a microorganism in which the ability to produce a sugar nucleotide from a nucleotide precursor and a sugar is acquired or improved by usual mutagenesis or the like can also be used as the microorganism for use in producing the sugar nucleotide according to the present invention.

Culturing of the microorganism of the present invention can be carried out in accordance with the usual culturing method.

The medium for use in the culturing of the microorganism may be either a nutrient medium or a synthetic medium, provided that it contains carbon sources, nitrogen sources, inorganic salts and the like which can be assimilated by the microorganism and it does not interfere efficient culturing of the microorganism.

Examples of the carbon sources include those which can be assimilated by each microorganism, such as carbohydrates (for example, glucose, fructose, sucrose, lactose, maltose, mannitol, sorbitol, molasses, starch, starch hydrolysate, *etc*.), organic acids (for example, pyruvic acid, lactic acid, citric acid, fumaric acid, *etc*.), various amino acids (for example, glutamic acid, methionine, lysine, *etc*.), and alcohols (for example, ethanol, propanol, glycerol, *etc*.). Also useful are natural organic nutrient sources, such as rice bran, cassava, bagasse, corn steep liquor, and the like.

Examples of the nitrogen sources include various inorganic and organic ammonium salts (for example, ammonia, ammonium chloride, ammonium sulfate, ammonium carbonate, ammonium acetate, ammonium phosphate, *etc*.), amino acids (for example, glutamic acid, glutamine, methionine, *etc*.), peptone, NZ amine, corn steep liquor, meat extract, yeast extract, malt extract, casein hydrolysate, soybean meal, fish meal or a hydrolysate of fish meal, and the like.

Examples of the inorganic salts include potassium dihydrogen phosphate, dipotassium hydrogen phosphate, disodium hydrogen phosphate, magnesium phosphate, magnesium sulfate, magnesium chloride, sodium chloride, calcium chloride, ferrous sulfate, manganese sulfate, copper sulfate, zinc sulfate, calcium carbonate, and the like.

Vitamins, amino acids, nucleic acids and the like may be added as occasion demands.

The culturing is carried out under aerobic conditions by shaking, aeration agitation culture or the like means. The culturing temperature is preferably from 15 to 45°C, and the culturing time is generally from 5 to 100 hours. The pH of the medium is maintained at 3 to 9 during the culturing. As occasion demands, adjustment of the pH of the medium is carried out using an inorganic or organic acid, an alkali solution, urea, calcium carbonate, ammonia, a pH buffer solution, and the like.

Also, antibiotics (for example, ampicillin, tetracycline, *etc*.) may be added to the medium during the culturing as occasion demands.

The microbial culture broth or a treated product of the culture broth obtained by treating the culture broth in various way can be used as an enzyme source for forming a sugar nucleotide in an aqueous medium.

Examples of the treated product of the culture broth include a concentrated product of the culture broth, a dried product of the culture broth, cells (microbial cells) obtained by centrifuging the culture broth, a dried product of the cells, a freeze-dried product of the cells, a surfactant-treated product of the cells, an ultrasonic-treated product of the cells, a mechanically disrupted product of the cells, a solvent-treated product of the cells, an enzyme-treated product of the cells, a protein fraction of the cells, an immobilized product of the cells and an enzyme preparation obtained by extracting from the cells.

Alternatively, commercially available microbial cells, dried microbial cells or the like may be used without culturing as the enzyme source for forming a sugar nucleotide. Examples of the enzyme source include commercially available cells of baker's yeast, beer yeast, and the like.

The amount of the enzyme source used in the formation of the sugar nucleotide is within the range of from 10 to 800 g/l, preferably from 50 to 600 g/l, as wet cells.

Examples of the aqueous medium used in the formation of the sugar nucleotide include water, buffer solutions (for example, those of phosphate, carbonate, acetate, borate, citrate, Tris, *etc*.), alcohols (for example, methanol, ethanol, *etc*.), esters (for example, ethyl acetate, *etc*.), ketones (for example, acetone, *etc*.), amides (for example, acetamide, *etc*.), and the like. The microbial culture broth used as the enzyme source may also be used as the aqueous medium.

Examples of the nucleotide precursor used in the formation of the sugar nucleotide include orotic acid, uracil, orotidine, uridine and the like. Preferred is orotic acid. The nucleotide precursor may be in the form of a purified product or in the form of a salt of the precursor, and a culture broth containing the precursor produced by the fermentation of a microorganism or the precursor roughly purified from the culture broth may also be used as the nucleotide precursor, provided that its impurities do not inhibit the reaction. The nucleotide precursor is used at a concentration of from 0.01 to 1.0 M, preferably from 0.01 to 0.3 M.

Examples of the sugar used in the formation of the sugar nucleotide include glucose, galactose, glucosamine, N-acetylglucosamine, and the like.

The sugar may be either in the form of a purified product or in the form of a material containing the same, with the proviso that impurities in the material do not inhibit the reaction, and is used at a concentration of from 0.01 to 1.0 M.

In the formation of the sugar nucleotide, an energy source necessary for the regeneration of ATP, a phosphate ion, a magnesium ion, a surfactant and an organic solvent may be added as occasion demands.

Examples of the energy source include carbohydrate (for example, glucose, fructose, sucrose, lactose, maltose, mannitol, sorbitol, *etc*.), organic acids (for example, pyruvic acid, lactic acid, acetic acid, *etc*.), amino acids (for example, glycine, alanine, aspartic acid, glutamic acid , *etc*.), molasses, starch hydrolysate, and the like, which may be used at a concentration of from 0.02 to 2.0 M.

Examples of the phosphate ion include orthophosphoric acid, polyphosphoric acids (for example, pyrophosphoric acid, tripolyphosphoric acid, tetrapolyphosphoric acid, tetrapolymetaphosphoric acid, *etc*.), polymetaphosphoric acids, inorganic phosphates (for example, potassium dihydrogen phosphate, dipotassium hydrogen phosphate, sodium dihydrogen phosphate, disodium hydrogen phosphate, *etc*.), and the like, which may be used at a concentration of from 0.01 to 1.0 M.

Examples of the magnesium ion include inorganic magnesium salts (for example, magnesium sulfate, magnesium nitrate, magnesium chloride, *et*c.), organic magnesium salts (for example, magnesium citrate*, etc*.), and the like, which may be used at a concentration of generally from 1 to 20 mM.

Examples of the surfactant include those which can enhance various sugar nucleotides, such as nonionic surfactants (for example, polyoxyethylene octadecylamine (e.g., Nymeen S-215, manufactured by Nippon Oil and Fats Co.), *etc*.), cationic surfactants (for example, cetyl trimethylammonium bromide, alkyldimethyl benzylammonium chloride (e.g., Cation F2-40E, manufactured by Nippon Oil and Fats Co.) *etc*.), anionic surfactants (for example, lauroyl sarcosinate, *etc*.) and tertiary amines (for example, alkyldimethylamine (e.g., Tertiary Amine FB, manufactured by Nippon Oil and Fats Co.), *etc*.), which may be used alone or as a mixture of two or more. The surfactant may be used at a concentration of generally from 0.1 to 50 g/l, preferably from 1 to 20 g/l.

Examples of the organic solvent include xylene, toluene, aliphatic alcohol, acetone, ethyl acetate, and the like, which may be used at a concentration of generally from 0.1 to 50 ml/l, preferably from 1 to 20 ml/l.

The reaction for forming a sugar nucleotide can be carried out in an aqueous medium at a pH value of from 5 to 10, preferably from 6 to 8, at a temperature of from 20 to 50°C and for a period of from 2 to 48 hours.

The sugar nucleotide can be formed by the method, and a uridine diphosphate compound can be exemplified as the sugar nucleotide. Specific examples include UDP-Glc, UDP-Gal, UDP-GlcNAc, and the like.

Determination of the sugar nucleotide formed in the aqueous medium can be carried out in accordance with a known method, for example, isolation and determination of UDP-Glc and UDP-Gal can be carried out by the high performance liquid chromatography (referred to as "HPLC" hereinafter) method described in *Anal. Biochem.*, 216, 188-194 (1994). In addition, isolation and determination of UDP-GlcNAc can be carried out by HPLC under the following conditions:

### Elution solution:

0.1 M KH₂PO₄ (adjust to pH 3.2 with H₃PO₄)

### Flow rate:

1 ml/min

### Column:

Partisil-10 SAX (manufactured by Whatman)

### Detection:

UV 262 nm

### Determination:

Calculated by comparing standard absorbance values

Recovery of the sugar nucleotide formed in the reaction solution can be carried out in the usual way using activated carbon, an ion exchange resin, and the like (Japanese Published Unexamined Patent Application No. 23993/96). For example, UDP-Gal and UDP-Glc can be recovered in accordance with the method described in *J. Org. Chem.*, 57, 152 (1992), and UDP-GlcNAc with the method described in *J. Org. Chem.*, 57, 146 (1992).

With regard to the microorganisms or animal cells used in producing the complex carbohydrate of the present invention, all microorganisms or animal cells capable of producing the complex carbohydrate from a sugar nucleotide and a complex carbohydrate precursor can be used. Examples thereof include animal cells or microorganisms, such as human melanoma cell line WM266-4 which produces β1,3-galactosyltransferase (ATCC CRL 1676), a recombinant line, such as namalwa cell line KJM-1 which contains the β1,3-galactosyltransferase gene derived from the human melanoma cell line WM266-4 (Japanese Published Unexamined Patent Application No. 181759/94), *Escherichia coli* which expresses the ceramide glucosyltransferase gene derived from human melanoma cell line SK-Mel-28 (*Proc. Natl. Acad. Sci. USA*, 93, 4638 (1996)), *Escherichia coli* (*EMBO J*., 9, 3171 (1990)) or *Saccharomyces cerevisiae* (*Biochem, Biophys. Res. Commun.*, 201, 160 (1994)) which expresses the β1,4-galactosyltransferase gene derived from human HeLa cells, COS-7 cell line (ATCC CRL 1651) which expresses the rat β1,6-N-acetylglucosaminyltransferase gene (*J. Biol. Chem.*, 268, 15381 (1993)), and the like.

When a microorganism is used for producing the complex carbohydrate of the present invention, the microorganism can be cultured using the same medium under the same culture conditions as in the case of the above-described microorganism capable of producing a sugar nucleotide from a nucleotide precursor and a sugar.

When animal cells are used for producing the complex carbohydrate of the present invention, the preferred culture medium is generally RPMI 1640 medium, Eagle's MEM medium or a medium thereof modified by further adding fetal calf serum, and the like. The culturing is carried out under certain conditions, for example, in the presence of 5% CO₂. The culturing is carried out at a temperature of preferably 35 to 37°C for a period of generally from 3 to 7 days. As occasion demands, antibiotics (for example, kanamycin, penicillin, *etc*.) may be added to the medium during the culturing.

The culture broth of a microorganism or an animal cell line obtained by the culturing and a treated product of the culture broth obtained by treating the culture broth in various ways can be used as enzyme sources for forming the complex carbohydrate in an aqueous medium.

Examples of the treated product of the culture broth include a concentrated product of the culture broth, a dried product of the culture broth, cells (microbial cells) obtained by centrifuging the culture broth, a dried product of the cells, a freeze-dried product of the cells, a surfactant-treated product of the cells, an organic solvent-treated product of the cells, a lytic enzyme-treated product of the cells, an immobilized product of the cells, an enzyme preparation obtained by extracting from the cells, and the like.

The enzyme source for forming the complex carbohydrate is typically used within the range of from 0.01 U/l to 100 U/l, preferably from 0.1 U/l to 100 U/l (where 1 unit (U) is the amount of the enzyme activity which can form 1 µM of the complex carbohydrate within 1 minute at 37°C).

Examples of the aqueous medium used in the formation of the complex carbohydrate include water, buffer solutions (for example, those of phosphate, carbonate, acetate, borate, citrate, Tris, *etc*.), alcohols (for example, methanol, ethanol, *etc*.), esters (for example, ethyl acetate, *etc*.), ketones (for example, acetone, *etc*.), amides (for example, acetamide, *etc*.), and the like. The microbial culture broth used as the enzyme source may also be used as the aqueous medium.

As the sugar nucleotide used in the formation of the complex carbohydrate, the above-mentioned reaction solution obtained by the sugar nucleotide formation or the sugar nucleotide recovered from the reaction solution can be used at a concentration of from 1 to 100 mM, preferably from 5 to 100 mM.

Examples of the complex carbohydrate precursor used in the formation of the complex carbohydrate include monosaccharides, oligosaccharides, proteins, peptides, glycoproteins, glycolipids and glycopeptides. Specific examples include N-acetylglucosamine, GlcNAcβ1-3Galβ1-4Glc, and the like. The complex carbohydrate precursor can be used at a concentration of from 0.1 to 100 mM, preferably from 0.5 to 50 mM.

Various complex carbohydrates can be formed by the method. Examples of the complex carbohydrates include glucose-containing complex carbohydrates, glucosamine-containing complex carbohydrates, galactose-containing complex carbohydrates, galactosamine-containing complex carbohydrates, mannose-containing complex carbohydrates, fucose-containing complex carbohydrates, neuraminic acid-containing complex carbohydrates, and the like. Specific examples include lacto-N-tetraose, lacto-N-neotetraose, N-acetyllactosamine, and the like.

In forming the complex carbohydrate, inorganic salts (for example, MnCl₂, *etc*.), β-mercaptoethanol, and the like can be added as occasion demands.

Determination of the complex carbohydrate formed in the aqueous medium can be carried out in accordance with the known method (Japanese Published Unexamined Patent Application No. 181759/94).

Recovery of the complex carbohydrate formed in the reaction solution can be carried out in the usual way using activated carbon, an ion exchange resin, and the like (Japanese Published Unexamined Patent Application No. 23993/96), for example, N-acetyllactosamine can be recovered in accordance with the method described in *J. Org. Chem.*, 47, 5416 (1982).

Examples of the present invention are given below by way of illustration and not by way of limitation.

### BEST MODE OF CARRYING OUT THE INVENTION

### Example 1: Production of UDP-Glc

A 2.5 L portion of a reaction solution having a composition of 100 g/l glucose, 1 g/l MgSO₄·7H₂O, 35 g/l K₂HPO₄ and 3 g/l orotic acid (potassium salt) was charged in a 5 L-culture vessel, a commercial baker's yeast (Dia Yeast; manufactured by Kyowa Hakko Kogyo Co., Ltd.) which had been subjected to a drying treatment was suspended in the reaction solution to a concentration of 100 g/l (dry weight basis), and the reaction was carried out for 6 hours at 28°C, 600 rpm of agitation and 2 L/min of aeration.

During the reaction, the pH of the reaction solution was maintained at 6.5 to 7.5 using 4 N KOH.

After completion of the reaction, amount of UDP-Glc in the supernatant of the reaction solution was measured by the method described in *Anal. Biochem*., 216, 188-194 (1994) to find that 7.4 g/l UDP-Glc (as 2 Na salt) was formed.

Microbial cells were removed from the reaction solution by centrifugation, and UDP-Glc was recovered from the thus obtained 2 L of supernatant in accordance with the method described in Japanese Published Unexamined Patent Application No. 23993/96, thereby obtaining an eluate containing high purity UDP-Glc.

After concentration of the eluate, excess amount of 99% ethanol was added thereto, and the thus formed precipitate was dried in vacuo to obtain 6.8 g of white powder. The powder was high purity (97% or more in purity) UDP-Glc.

### Example 2: Production of UDP-Gal

*Kluyveromyces marxianus* var*. bulugaricus* ATCC 16045 line was inoculated into a 300 ml-conical flask containing 20 ml of an aqueous medium composed of 50 g/l glucose, 2 g/l yeast extract, 5 g/l peptone, 2 g/l (NH₄)₂HPO₄, 2 g/l KH₂PO₄ and 1 g/l MgSO₄·7H₂O (adjusted to pH 6.0 with 6 N H₂SO₄) and then cultured at 28°C for 24 hours under shaking at 220 rpm. The thus obtained culture broth was used as the first seed culture.

A 20 ml portion of the seed culture was added to a 2 L-baffled conical flask containing 240 ml of an aqueous medium composed of 50 g/l lactose, 2 g/l yeast extract, 5 g/l peptone, 2 g/l (NH₄)₂HPO₄, 2 g/l KH₂PO₄ and 1 g/l MgSO₄·7H₂O (adjusted to pH 6.0 with 6 N H₂SO₄) and then cultured at 28°C for 24 hours under shaking at 220 rpm. The thus obtained culture broth was used as the second seed culture.

A 250 ml portion of the second seed culture was inoculated into 2.5 L of an aqueous medium having a composition of 100 g/l lactose, 2 g/l yeast extract, 5 g/l peptone, 2 g/l (NH₄)₂HPO₄, 2 g/l KH₂PO₄ and 1 g/l MgSO₄·7H₂O (adjusted to pH 6.0 with 6 N H₂SO₄) which had been charged in a 5 L-culture vessel, and then the culturing was carried out for 24 hours at 28°C, 600 rpm of agitation and 2.5 L/min of aeration.

During the culturing, the pH of the culture broth was maintained at 5.5 (± 0.1) using 28% aqueous ammonia.

After completion of the culturing, 4 g/l Nymeen S-215, 3 g/l orotic acid (potassium salt), 1 g/l magnesium sulfate, 3 g/l KH₂PO₄, 4 g/l K₂HPO₄ and 18 g/l galactose were added to the culture broth, and then the reaction was carried out for 15 hours at 28°C, 600 rpm of agitation and 2.0 L/min of aeration.

During the reaction, the pH of the reaction solution was maintained at pH 6.0 to 7.0 using 4 N KOH.

After completion of the reaction, the amount of UDP-Gal in the supernatant was measured by the method described in *Anal. Biochem.*, 216, 188-194 (1994) to find that 2.3 g/l of UDP-Gal (as 2 Na salt) was formed.

Microbial cells were removed from the reaction solution by centrifugation, and the resulting 2 L of supernatant was subjected to purification in the same manner as in Example 1 to obtain 2.5 g of white powder of high purity (97% or more in purity) UDP-Gal.

### Example 3: Production of UDP-GlcNAc

Production of UDP-GlcNAc was carried out under the same conditions as in Example 1, except that 100 g/l maltose was used in stead of glucose, and 3.5 g/l glucosamine hydrochloride was newly added to the reaction solution.

After completion of the reaction, amount of UDP-GlcNAc in the reaction solution supernatant was measured by the above-mentioned HPLC method to find that 7 g/l UDP-GlcNAc (as 2 Na salt) was formed.

Microbial cells were removed from the reaction solution by centrifugation, and the resulting 2 L of supernatant was subjected to purification in the same manner as in Example 1 to obtain 5.7 g of white powder of high purity (97% or more in purity) UDP-GlcNAc.

### Example 4: Preparation of β1,3-galactosyltransferase

A namalwa line KJM-1 transformed with a plasmid pAMoERSAW1 (Japanese Published Unexamined Patent Application No. 181759/94) containing a gene encoding a fusion protein of the IgG binding region of protein A with β1,3-galactosyltransferase was suspended in 30 ml of RPMI 1640 ITPSGF medium containing 0.5 mg/ml G418 (manufactured by Gibco) to a density of 5×10⁴ cells/ml and cultured at 37°C for 8 days in a CO₂ incubator.

Cells were removed from the culture broth by centrifugation, and the resulting supernatant was recovered. If necessary, the supernatant can be stored at -80°C and used by thawing it prior to use.

To the culture supernatant in which the fusion protein of the IgG binding region of protein A with β1,3-galactosyltransferase has been formed were added sodium azide to a final concentration of 0.1% and then 50 µl of IgG Sepharose (manufactured by Pharmacia) which has been pretreated in accordance with the manufacturer's instructions, subsequently stirring the mixture overnight gently at 4°C.

After stirring, the β1,3-galactosyltransferase-linked IgG Sepharose was recovered by centrifugation and washed three times with 1 ml of RPMI 1640 ITPSGF medium, and the IgG Sepharose was used as the enzyme source of β1,3-galactosyltransferase.

### Example 5: Production of lacto-N-tetraose

Lacto-N-neotetraose (manufactured by Oxford Glycosystems) was fluorescence-labeled with aminopyridine in accordance with the conventional method (*Agric. Biol. Chem*., 54, 2169 (1990)) and then mixed with 100 mU of β-galactosidase (manufactured by Seikagaku Kogyo K.K.) to carry out 16 hours of reaction at 37°C, thereby removing galactose at the non-reducing end.

The reaction solution was heated at 100°C for 5 minutes to inactivate β-galactosidase.

GlcNAcβ1-3Galβ1-4Glc obtained by the reaction was used as a complex carbohydrate precursor.

A 36 µl portion of a reaction solution containing 0.5 mM of the complex carbohydrate precursor, 0.5 U of the β1,3-galactosyltransferase linked IgG Sepharose obtained in Example 4, 5 mM of UDP-Gal obtained in Example 2, 100 mM of Tris-HCl (pH 7.9), 10 mM of MnCl₂ and 2 mM of β-mercaptcethanol was allowed to stand for 65 hours at 32°C to effect the reaction.

After completion of the reaction, amount of the product accumulated in the reaction solution was measured by HPLC under the following conditions:

### Column:

TSK gel ODS-80TM column

(4.6 mm × 30 cm, manufactured by TOSOH Corporation)

### Liquid phase:

0.02 M Ammonium acetate buffer (pH 4.0)

### Temperature:

50°C

### Flow rate:

1 ml/min

### Detection:

Fluorescence detector (excitation wave length 320 nm, radiation wave length 400 nm)

Identification of the product was carried out by comparing elution time of aminopyridine-labeled lacto-N-tetraose with that of the labeled product.

By the reaction, 0.17 mM (0.12 g/l) of lacto-N-tetraose was formed.

### Example 6: Production of lacto-N-neotetraose

A 36 µl portion of a reaction solution containing 0.5 mM of the complex carbohydrate precursor GlcNAcβ1-3Galβ1-4Glc prepared in Example 5, 0.5 U β1,4-galactosyltransferase (manufactured by Sigma), 5 mM UDP-Gal obtained in Example 2, 100 mM Tris-HCl (pH 7.9), 10 mM MnCl₂, 2 mM β-mercaptoethanol and 0.2 mg/ml of α-lactoalbumin was allowed to stand for 65 hours at 32°C to effect the reaction.

After completion of the reaction, the amount of the product accumulated in the reaction solution was measured under the same conditions as in Example 5 with HPLC. Identification of the product was carried out by comparing elution time of aminopyridine-labeled lacto-N-neotetraose with that of the labeled product.

By the reaction, 0.2 mM (0.14 g/l) of lacto-N-neotetraose was formed.

### INDUSTRIAL APPLICABILITY

The present invention renders possible efficient industrial production of a sugar nucleotide from a nucleotide precursor and a sugar and of a complex carbohydrate from the sugar nucleotide and a complex carbohydrate precursor.

## Claims

1. A process for producing a complex carbohydrate, which comprises:
selecting, as enzyme sources, a culture broth of a microorganism capable of producing a sugar nucleotide from a nucleotide precursor and a sugar, or a treated product of the culture broth, and a culture broth of a microorganism or animal cell capable of producing a complex carbohydrate from a sugar nucleotide and a complex carbohydrate precursor, or a treated product of the culture broth;
carrying out an enzyme reaction in an aqueous medium containing the enzyme sources, the nucleotide precursor, the sugar and the complex carbohydrate precursor to form and accumulate the complex carbohydrate in the aqueous medium; and
recovering the complex carbohydrate from the aqueous medium.

2. A process for producing a sugar nucleotide, which comprises:
selecting, as an enzyme source, a culture broth of a microorganism capable of producing a sugar nucleotide from a nucleotide precursor and a sugar, or a treated product of the culture broth;
carrying out an enzyme reaction in an aqueous medium containing the enzyme source, the nucleotide precursor and the sugar to form and accumulate the sugar nucleotide in the aqueous medium; and
recovering the sugar nucleotide from the aqueous medium.

3. A process for producing a complex carbohydrate, which comprises:
selecting, as an enzyme source, a culture broth of a microorganism or animal cell capable of producing a complex carbohydrate from a sugar nucleotide and a complex carbohydrate precursor, or a treated product of the culture broth;
carrying out an enzyme reaction in an aqueous medium containing the enzyme source, the complex carbohydrate and the sugar nucleotide prepared according to the process of claim 2 to form and accumulate the complex carbohydrate in the aqueous medium, and
recovering the complex carbohydrate from the aqueous medium.

4. The process according to any one of claims 1, 2 and 3, wherein the treated product of the culture broth is a concentrated product of the culture broth, a dried product of the culture broth, cells obtained by centrifuging the culture broth, a dried product of the cells, a freeze-dried product of the cells, a surfactant-treated product of the cells, an ultrasonic-treated product of the cells, a mechanically disrupted product of the cells, a solvent-treated product of the cells, an enzyme-treated product of the cells, a protein fraction of the cells, an -mobilized product of the cells or an enzyme preparation obtained by extracting from the cells.

5. The process according to claim 1 or 2, wherein the nucleotide precursor is a nucleotide precursor selected from orotic acid, uracil, orotidine and uridine.

6. The process according to any one of claims 1, 2 and 3, wherein the sugar nucleotide is a uridine diphosphate compound.

7. The process according to claim 6, wherein the uridine diphosphate compound is a uridine diphosphate compound selected from uridine-diphosphate glucose, uridine-diphosphate galactose, uridine-diphosphate N-acetylglucosamine and uridine-diphosphate N-acetylgalactosamine.

8. The process according to claim 1 or 2, wherein the sugar is a sugar selected from glucose, galactose, glucosamine, N-acetylglucosamine and N-acetylgalactosamine.

9. The process according to claim 1 or 3, wherein the complex carbohydrate precursor is a complex carbohydrate precursor selected from monosaccharides, oligosaccharides, proteins, peptides, glycoproteins, glycolipids and glycopeptides.

10. The process according to claim 9, wherein the complex carbohydrate precursor is N-acetylglucosamine or GlcNAcβ1-3Galβ1-4Glc.

11. The process according to claim 1 or 3, wherein the complex carbohydrate is a glucose-containing complex carbohydrate, a glucosamine-containing complex carbohydrate, a galactose-containing complex carbohydrate, a galactosamine-containing complex carbohydrate, a mannose-containing complex carbohydrate, a fucose-containing complex carbohydrate or a neuraminic acid-containing complex carbohydrate.

12. The process according to claim 11, wherein the galactose-containing complex carbohydrate is a complex carbohydrate selected from lacto-N-tetraose and lacto-N-neotetraose.

13. The process according to claim 1 or 2, wherein the microorganism capable of producing a sugar nucleotide from a nucleotide precursor and a sugar is a yeast.

14. The process according to claim 13, wherein the yeast is a yeast selected from microorganisms belonging to the genus *Saccharomyces*, the genus *Candida*, the genus *Pichia*, the genus *Torulopsis*, the genus *Debaryomyces*, the genus *Zygosaccharomyces*, the genus *Kluyveromyces*, the genus *Hansenula* and the genus *Brettanomyces*.

15. The process according to claim 14, wherein the yeast is a yeast selected from *Saccharomyces cerevisiae*, *Candida utilis*, *Candida parapsilosis*, *Candida krusei*, *Candida versatilis*, *Candida lipolytica*, *Candida zeylanoides*, *Candida guilliermondii*, *Candida albicans*, *Candida humicola*, *Pichia farinosa*, *Pichia ohmeri*, *Torulopsis candida*, *Torulopsis sphaerica, Torulopsis xylinus*, *Torulopsis famata*, *Torulopsis versatilis*, *Debaryomyces subglobosus*, *Debaryomyces cantarellii*, *Debaryomyces globosus*, *Debaryomyces hansenii*, *Debaryomyces japonicus*, *Zygosaccharomyces rouxii*, *Zygosaccharomyces bailii*, *Kluyveromyces marxianus*, *Hansenula anomala, Hansenula jadinii*, *Brettanomyces lambicus* and *Brettanomyces anomalus*.

16. The process according to claim 1 or 3, wherein the microorganism capable of producing a complex carbohydrate from a sugar nucleotide and a complex carbohydrate precursor is *Escherichia coli* or *Saccharomyces cerevisiae*.

17. The process according to claim 1 or 3, wherein the animal cell capable of producing a complex carbohydrate from a sugar nucleotide and a complex carbohydrate precursor is COS-7 cell or namalwa KJM-1 cell.

18. The process according to claim 17, wherein the namalwa KJM-1 cell is a namalwa KJM-1 cell which contains a recombinant DNA of a DNA fragment containing a gene encoding β1,3-galactosyltransferase with a vector.

19. The process according to claim 18, wherein the gene encoding β1,3-galactosyltransferase is derived from human melanoma cells.

20. The process according to claim 18, wherein the animal cell is namalwa KJM-1/pAMoERSAW1.
